# EUROPÄISCHE PATENTSCHRIFT

(11) **EP 1 660 033 B1**
(45) Veröffentlichungstag und Bekanntmachung des Hinweises auf die Patenterteilung: **10.11.2010**
(21) Anmeldenummer: 04764494.3
(22) Anmeldetag: 26.08.2004
(51) Int. Cl.: A61K 8/99, A61K 8/18

(54) **KOSMETISCHE KUGELFÖRMIGE ZUBEREITUNGEN ZUR TOPISCHEN ANWENDUNG**
SPHERICAL COSMETIC PREPARATIONS FOR TOPICAL APPLICATION
PREPARATIONS COSMETIQUES SOUS FORME SPHERIQUE POUR APPLICATION TOPIQUE

(30) Priorität: 27.08.2003 DE 10339747; 30.08.2003 DE 10340106; 10.12.2003 DE 10357640
(43) Veröffentlichungstag der Anmeldung: 31.05.2006
(73) Patentinhaber: Beiersdorf AG, 20253 Hamburg (DE)
(72) Erfinder: RASCHKE, Thomas, 25421 Pinneberg (DE); HETZEL, Frank, 21261 Welle (DE); KALLMAYER, Volker, 22393 Hamburg (DE)
(86) Internationale Anmeldenummer: PCT/EP2004/009518
(87) Internationale Veröffentlichungsnummer: WO 2005/020949

(56) Entgegenhaltungen:
- EP-A- 0 234 078
- EP-A- 1 201 219
- EP-A- 1 473 016
- WO-A-00/10522
- WO-A-01/03538
- DE-A- 4 223 004
- DE-A- 10 044 062
- DE-A- 10 209 111
- US-A1- 2002 022 038
- DATABASE WPI Section Ch, Week 199837 Derwent Publications Ltd., London, GB; Class A26, AN 1998-433672 XP002313336 & JP 10 182337 A (NOEVIR KK) 7. Juli 1998 (1998-07-07)
- DATABASE WPI Section Ch, Week 200355 Derwent Publications Ltd., London, GB; Class A14, AN 2003-580900 XP002313041 & JP 2003 073230 A (SHISEIDO CO LTD) 12. März 2003 (2003-03-12)
- DATABASE WPI Section Ch, Week 200227 Derwent Publications Ltd., London, GB; Class A96, AN 2002-211272 XP002313250 & JP 2001 348310 A (KANEBO LTD) 18. Dezember 2001 (2001-12-18)

## Beschreibung

Die Erfindung umfasst eine kosmetische und/oder dermatologische Zubereitung zur topischen Anwendung auf Basis einer wasserhaltigen Emulsion in Form von festen, halbfesten und/oder formstabilen Kugeln, Sphäroiden oder anders geformten Körpern enthaltend im Wesentlichen Wachse, Emulgatoren, natürliche und/oder synthetische Polymere und/oder Mischungen daraus. Die Zubereitung schmilzt beim Verreiben und/oder Verteilen auf der Haut und/oder dem Haar und/oder wird aufgrund von Scherkräften völlig oder teilweise flüssig und/oder vermischt sich mit den Lipiden des Hautsebums und/oder löst sich darin.

Tabletten zur peroralen pharmazeutischen Anwendung sind seit spätestens dem 19. Jahrhundert bekannt. Es handelt sich hierbei um einzeldosierte feste Arzneiformen, die aus trockenen Pulvern, Kristallen oder Granulaten unter hohem Druck und in Gegenwart von Hilfsstoffen geformt werden (Rudolf Voigt, "Pharmazeutische Technologie", Deutsche Apotheker Verlag, Stuttgart, 9. Auflage (2000), S. 163 ff.).
Auch Suppositorien zur rektalen oder vaginalen Anwendung werden in der Pharmazie seit langem verwendet. Hierbei handelt es sich um formbeständige Zubereitungen, die bei Körpertemperatur schmelzen oder sich in wässrigem Milieu langsam auflösen (Rudolf Voigt, "Pharmazeutische Technologie", Deutsche Apotheker Verlag, Stuttgart, 9. Auflage (2000), S. 267 ff.). Sie können durch Gießen oder Pressen hergestellt werden. Je nach Herstellverfahren werden neben den üblichen pharmazeutischen Anforderungen wie gute Verträglichkeit und Kompatibilität mit dem Wirkstoff noch Haltbarkeit weitere Anforderungen an die verwendeten Rohstoffe gestellt, wie z.B. geringe Unterschiede zwischen Schmelz- und Erstarrungspunkt sowie zwischen Fließ- und Klarschmelzpunkt und zumindest kinetische Stabilität der zuerst gebildeten Kristallmodifikation. Suppositorien zur topischen Anwendung sind bisher nicht beschrieben worden.
Aus dem Stand der Technik sind darüber hinaus pharmazeutische Kapseln, beispielsweise Weich- oder Hartgelantinekapseln bekannt, die aus einer Hülle aus Gelantine und Glycerol sowie gelegentlich Farbstoffen, und einer flüssigen, pastösen oder partikulären Füllung bestehen. Das Verfahren zur Herstellung dieser seit vielen Jahren bekannten Kapseln erfolgt z.B. nach dem sog. Rotary-Die-System (Seifen-Öle-Fette-Wachse, 113. Jg; Nr 3/1987, Seite 67 ff.). Diese Gelantinekapseln lösen sich beim Verschlucken im Magen-Darmtrakt auf und geben ihre Inhaltstoffe frei. Da es ohne Weiteres möglich ist, Kapseln in unterschiedlichen Größen und Formen zu erzeugen, ist die Anwendung nicht nur auf auf perorale Applikationsformen beschränkt (Rudolf Voigt, "Pharmazeutische Technologie", Deutsche Apotheker Verlag, Stuttgart, 9. Auflage (2000), S. 543 ff.). Eine orale Resorption kann durch Lutschkapseln erzielt werden. Sie sind innen hohl und besitzen eine dreifach stärkere Wanddicke als andere Kapseln. Der Wirkstoff ist hierbei in der Gelantinehülle eingearbeitet. Ein weiteres Beispiel sind Nitroglycerin-Kaukapsein (Zerbeißkapsein), die ebenfalls eine rasche Resorption des Wirkstoffs über die Mundschleimhaut ermöglichen. Desweiteren können einzeldosierte Arzneistoffe nach Aufstechen oder Aufschneiden der tubenförmigen Salbenkapseln durch Ausquetschen des Inhalts zur Applikation gebracht werden (perkutane Applikation von Nitroglycerjn-Herzsalbe).

Aus dem Lebensmittelsbereich sind beispielsweise verschiedene, bei Raumtemperatur feste Zubereitungen bekannt, die bei Körpertemperatur schmelzen. Es handelt sich hierbei meistens um Pflanzenfettzubereitungen, besonders auf Basis von Kakaobutter sowie Sheabutter (Schokolade) oder W/O-Emulsionen aus gehärteten Pflanzenfetten und freien Fettsäuren (Margarine). Als "Eiskonfekt" sind Zubereitungen bekannt, bei denen eine betragsmäßig hohe Schmelzenthalpie aufgebracht werden muss und die daher im Mund einen Kühleffekt haben.
Darüber hinaus sind Pralinees bekannt, die in einer formstabilen Schokoladenhülle flüssige oder pastöse Inhaltsstoffe enthalten. Die Hülle schmilzt beim Lutschen oder Zerbeißen im Mund oder nach dem Verschlucken.

Im Kosmetikbereich sind die sogenannten Badeperlen bekannt, deren Hülle, z.B aus Gelatine sich im heißen oder warmen Badewasser rückstandsfrei auflöst und ihren Inhalt, beispielsweise Tensidzubereitungen, Emulsionen, Lipide, Farbstoffe und/oder Parfüme, ins Badewasser freigibt. Da die Hülle aus Gelatine besteht, darf das Produkt kein Wasser enthalten, andernfalls würde die Hülle während der Lagerung aufweichen.

Eine zweite Gruppe kosmetischer Kapseln umfasst alle Produkte, für welche die Kapselhülle nur ein Behältnis zur einmaligen Dosierung und Anwendung darstellt und deren Hülle nach der Anwendung übrig bleibt. Nachteilig ist hieran, dass die übrig bleibende Hülle störend wirkt und zudem entsorgt werden muss.

Zahlreiche kosmetische und/oder dermatologische Wirkstoffe sind gegenüber bestimmten Einflüssen wie Feuchtigkeit, niedrigen oder hohen pH-Werten sowie Sauerstoff oder Licht nicht stabil. Es hat daher nicht an Versuchen gefehlt, derartige Wirkstoffe den genannte unerwünschten Umwelteinflüssen so zu entziehen, dass dennoch bei der Anwendung eine Freisetzung der Wirkstoffe stattfindet. Ein Weg zur Erreichung dieses Ziels ist die Mikro- oder Nanoverkapselung von Wirkstoffen. Das Verkapselungsmaterial als Trägersystem für die Wirkstoffe erlaubt dabei, sie vor Umwelteinflüssen geschützt in geeignete Zubereitungen einzuarbeiten, ohne dass der Verwender die Kapseln bei der Produktapplikation wahrnehmen könnte.
Ziel einer solchen Verkapselung ist es beispielsweise, wirkstoffhaltige Wachspartikel im -Mikrometerbereich (1 - 250 µm) herzustellen, die in gängigen pastösen oder flüssigen kosmetischen Zubereitungen einzuarbeiten sind. Es ist bisher nicht bekannt, diese Mikrokapseln als eigenständige kosmetische Zubereitung herzustellen, zu lagern und topisch anzuwenden.

Zur Verkapselung kosmetischer Wirkstoffe gibt es mehrere Ansätze. Bekannt ist beispielsweise die liposomale Verkapselung von Arzneistoffen, die zu einer retardierten Wirkstoff-Freisetzung führen soll. Im Wesentlichen handelt es sich dabei um von Phospholipiden oder anderen Amphiphilen umschlossene wirkstoffhaltige sphärische Vesikel, die sogenannten Liposome. Die Langzeitstabilität derartiger Gebilde ist jedoch gering.
Nanopartikel sind feste Partikel mit Partikelgrößen von 20 bis 500 nm. Gelegentlich werden auch größere Teilchen mit Durchmessern bis zu 1000 nm zu den Nanopartikeln gerechnet. Derartige Partikel bestehen in der Regel aus Polymeren und weisen Kavitäten auf oder bilden eine Hülle, so dass sich in ihrem Inneren Gastmoleküle aufhalten können, die umschlossen oder adsorbiert werden. Diese Gastmoleküle werden dann bei der Anwendung des Nanopartikel enthaltenden Produktes langsam freigesetzt. Ähnlich verhalten sich feste Lipidnanopartikel, die in einer Matrix aus festen Lipiden verteilte Wirkstoffe enthalten. Die Größe der Partikel ist mit denen der Nanopartikel vergleichbar.

Zur Verkapselung von pharmazeutischen oder kosmetischen Wirkstoffen zur Kontrolle der Wirkstoff-Freisetzung oder der stabilen Einarbeitung in Zubereitungen sind zahlreiche Methoden bekannt.

Die europäische Patentanmeldung EP064911 bzw. EP 1064912 offenbart Wirkstoffe enthaltende Mikrokapseln mit einem Durchmesser von 0,1 bis 5 mm, die man erhält, indem man aus Gelbildnern, Chitosan und Wirkstoff eine Matrix herstellt, und diese in wässrige Lösungen anionischer Polymere eintropft. Dabei bildet sich aus Chitosan und anionischem Polymer eine Membran, die die Wirkstofflösung umhüllt. Diese Mikropartikel werden dann wiederum als Bestandteil gängiger kosmetischer Zubereitungen verwendet. Allgemeines zu Verkapselungstechniken mit Chitosan findet sich in Journal of Microencapsulation 14, Seiten 689-711 (1997).

In Lipidpartikeln verkapselte, zumeist lipophile Wirkstoffe sind an sich dem Fachmann bekannt. So beschreiben die EP 167825, DE 100 59 668, DE 199 45 203, EP 0934743, WO 94/20072, WO 00/10522 sowie die WO 00/67728 wirkstoffbeladene Lipidpartikel. Doch konnten diese Schriften nicht das Problem der Bereitstellung von kugelförmigen Zubereitungen mit bzw. ohne kosmetischen Inhaltsstoffen lösen, die als eigenständige kosmetische Zubereitung herzustellen, zu lagern und topisch anzuwenden sind.

Bei Raumtemperatur feste Lippenpflegestifte aus W/O-Emulsionen werden beispielsweise in der DE 10148313 beschrieben. Diese dort beschriebene Technologie ist hiermit Gegenstand der vorliegenden Erfindung. Die Emulsionen gemäß DE 10148313 werden beim Verteilen oder Verreiben auf der Haut nicht flüssig, es findet lediglich ein Abrieb der Emulsion auf dem Anwendungsareal, besonders den Lippen, statt. Eine Verflüssigung der dort beschriebenen Zubereitungen ist sogar unerwünscht, da zur Pflege der Lippen das aufgebrachte Material möglichst lange am Auftragungsort verweilen und nicht beispielsweise abfließen soll.

Aufgabe der vorliegenden Erfindung ist es, eine kosmetische Zubereitung bereit zu stellen, die als formstabile Kugel vorliegt, damit einzeln portionierbar ist und als Ganzes auf der Haut verteilt werden kann. Dabei sollen keinerlei Rückstände, Kapselmaterial etc. zurückbleiben, die ggf. gesondert entsorgt werden müssten oder auf der Haut sichtbar blieben. Insbesondere ist es die Aufgabe der vorliegenden Erfindung eine kosmetische Zubereitung bereit zu stellen, die eine neue kosmetische Produktform darstellt und dem Anwender ein neues Applikationserlebnis bietet und das Anwendungsspektrum von Haut- und/oder Haarpflegeprodukten erweitert.

Gelöst wird dieses Bündel an Aufgaben durch eine kosmetische Zubereitung entsprechend den Hauptansprüchen. In den Unteransprüchen sind bevorzugte Ausführungsformen der Zubereitung offenbart. Die Erfindung umfasst darüber hinaus auch das Verfahren zur Applikation der Zubereitung auf der Haut und/oder dem Haar, die Verwendung und die Kombination mit einer Blisterverpackung und Schachteln.
Es war überraschend und für den Fachmann außerordentlich erstaunlich, dass die gestellten Aufgaben gelöst werden können durch eine kosmetische und/oder dermatologische Zubereitung auf Basis einer W/O-Emulsion in Form von bis zu einer Temperatur von 35°C festen, halbfesten und/oder formstabilen Kugeln oder Sphäroiden mit einem Volumen von 0,1 bis 20 ml enthaltend im wesentlichen Wachse, Lipide, Emulgatoren, natürliche und/oder synthetische Polymere und/oder Mischungen daraus, wobei die Zubereitung aus einer formstabilen Lipid-/Emulgatormischung besteht, die dispergiertes Wasser mit einer Tröpfchengröße unter 50 Mikrometer enthält. Unter Lagerbedingungen, d.h. Raumtemperatur oder niedriger, sind die Zubereitungen ganz oder teilweise fest.
Der Vorteil und gleichzeitig die erfindungsgemäße Eigenschaft der kapselförmigen Zubereitung ist, dass sie
- beim Verreiben und/oder Verteilen der Kapsel auf der Haut und/oder dem Haar schmilzt und/oder
- aufgrund von Scherkräften völlig oder teilweise flüssig wird und/oder
- sich in der Füllung und/oder den Hautsebumlipiden bzw. durch Vermischung von Füllung und Hüllmaterial auflöst
und so, insbesondere für den Anwender, nicht mehr, insbesondere als gesondert neben der Füllung vorliegende Bestandteil, wahrnehmbar ist.
D.h. die Kapsel zieht vorteilhafterweise bei der Applikation auf der Haut oder dem Haar vollständig ein ohne Rückstände zu hinterlassen
Insbesondere gegenüber den bekannten kosmetischen Kapseln, deren Kapselhülle nach der Anwendung übrig bleibt, kann erfindungsgemäß die hier beschriebene Kugel vollständig auf der Haut verbleiben. Damit kann die gesamte Kugel einen aktiven, pflegenden Beitrag in der kosmetischen und dermatologischen Zubereitung leisten.
Viele Begriffe wie "Kugeln" oder "Kapseln" können grundsätzlich zur Beschreibung der erfindungsgemäßen Zubereitung herangezogen werden, jedoch werden diesen Begriffen unter Umständen verschiedene Bedeutungen zugeordnet. So werden mit den Begriffen "Pralinée" oder "Suppositorium" jeweils andere, teilweise recht unkosmetische

Anwendungsarten verbunden. Der Begriff "Kugel" oder "Kapsel" zur Beschreibung der erfindungsgemäßen Zubereitung ist hierbei nicht auf die mathematische Bedeutung beschränkt, sondern umfasst alle exakt oder angenährt, regelmäßig oder unregelmäßig kugelförmigen, spärischen, runden, ovalen, ellipsoiden Körper ebenso wie solche mit Ecken und Kanten, also beispielsweise Würfel, Quader, Parallelepipede, Sterne, Herzen und dergleichen.

Die erfindungsgemäße Zubereitung umfasst bei Raumtemperatur (RT) formstabile vorportionierte kosmetische Produkte, die beim Verreiben und/oder Verteilen auf der Haut und/oder dem Haar schmelzen, flüssig werden oder sich auflösen. Damit ist gewährleistet, dass das Kosmetikum ohne störende Rückstände auf der Haut oder dem Haar appliziert werden kann.

Es verbleiben gegenüber dem Stand der Technik bekannten kapselförmigen Präparaten keinerlei Materialbestandteile ungenutzt auf der Haut zurück, die unansehnlich sind, unnötige Kosten und Umweltbelastungen verursachen und darüber hinaus nach der Produktapplikation vom Verbraucher entfernt werden müssen. Die dem Stand der Technik bekannten nicht kapselförmigen Präparate wie Schokolade oder Suppositorien sind nicht zur topischen Anwendung geeignet, da sie erstens eine ungeeignete Schmelztemperatur aufweisen (die Temperatur der Haut liegt mitunter wesentlich unter der normalen Körpertemperatur) und sich zweitens nicht verreiben lassen. Drittens handelt es sich (mit Ausnahme von Margarine) nicht um Emulsionen und durch das Fehlen von Wasser wird im Gegensatz zur vorliegenden Erfindung ein sehr unangenehmes, unkosmetisches Hautgefühl verursacht. Beispielsweise im Falle von Margarine steht einer Verwendung im Sinne der vorliegenden Erfindung ein Mangel an Formstabilität während der Lagerung entgegen.

Die erfindungsgemäßen Kugeln weisen eine Größe, d.h. einen durchschnittlichen Durchmesser, von 3 bis 40 mm auf, Die erfindungsgemäßen Zubereitungen können beliebige Formen haben. Sie sind jedoch sphärisch mit einem Volumen von 0,1 bis 20 ml. Damit sind die Kugeln einzeln handhab- und anwendbar. Die Zubereitungen sind als Dragees, Kugeln, Sphäroide, Ellipsoide oder anders geformte Körper bei Lagerung und Entnahme formstabil und werden erst beim Verteilen und/oder Verreiben auf der Haut und/oder dem Haar flüssig. Dies wird beispielsweise durch die spezielle Zusammensetzung der Lipidphase der W/O-Emulsion erreicht.

Die erfindungsgemäße kugelförmige Zubereitung muss unter Lagerungsbedingungen, denen kosmetische Produkte üblicherweise ausgesetzt sind, fest und formstabil sein, das heißt die Form der erfindungsgemäßen Kugeln darf sich während der Lagerung nicht durch beispielsweise den Einfluss der Gravitation oder der Temperatur bis zu 35°C verändern. Idealerweise kleben die Kugeln während der Lagerung auch dann nicht zusammen, wenn sich zwei Kugeln über längere Zeit berühren. Sollte dieses Erfordernis technologisch unpraktisch sein, lässt sich das Problem durch eine individuelle Umverpackung jeder einzelnen Kugel, ähnlich einer Einzelverpackung (Bonbon-Papier) lösen. Gegebenenfalls ist es vorteilhaft, die einzelnen Kugeln mit Hüllen zu überziehen, die die Formstabilität verbessern, das Zusammenkleben einzelner Kugeln verhindern oder verringern und/oder den Gewichtsverlust durch Verdunstung von Wasser und/oder anderer leicht flüchtiger Komponenten verringern oder verhindern.

Das Kugelmaterial der erfindungsgemäßen Zubereitung verbleibt nach der Anwendung vollständig auf der Haut ohne unahnsehnliche Rückstände zu hinterlassen. Die erfindungsgemäße Zubereitung zeigt somit auch vorteilhafte Eigenschaften bekannter kosmetischer Creme, Lotion etc. Erfindungsgemäß ist es von großer Bedeutung, dass alle Rohstoffe, aus denen die Kugel aufgebaut ist, sehr gut hautverträglich sind. Idealerweise leisten sie einen Beitrag zur Hautpflege, beispielsweise dadurch, dass sie die natürliche Hautbarriere stärken und die Haut so vor Austrocknung schützen.
Die Zusammensetzung der Kugel beeinflusst darüber hinaus maßgeblich das Hautgefühl des Verbrauchers bei Anwendung der erfindungsgemäßen Zubereitungen. Deshalb ist es vorteilhaft, die Kugel aus solchen Stoffen aufzubauen, die bei der Anwendung ein angenehmes Hautgefühl bewirken.
Die hier beschriebenen technischen und sensorischen Anforderungen an die erfindungsgemäße Zubereitung gehen deutlich über das übliche Anforderungsspektrum für bekannte kosmetische Zubereitungen hinaus. Deshalb ist es überraschend, dass sich erfindungsgemäße Zubereitung mit Rohstoffen, wie sie dem Fachmanne bereits bekannt sind und in der Kosmetik bereits eingesetzt werden, herstellen lassen.

Es sind folgende Prinzipien zu beachten.

Einerseits können Lipide, deren Schmelzpunkt nahe an der Hauttemperatur von 32°C, also zwischen 30°C und 40°C liegt, zur Herstellung einer solchen erfindungsgemäßen Zubereitung verwendet werden. Andererseits kann durch Verwendung geeigneter Wachse, polymerer Verdicker und/oder Gelbildner aus flüssigen Lipidsystemen eine thixotrope Zubereitung mit hoher Fließgrenze hergestellt werden, die den Anforderungen an die beschriebene Zubereitung genügt. Dem Fachmanne ist offensichtlich, dass die genannten Ansätze, Verwendung von Lipiden mit vorteilhaften Schmelzpunkten und Verwendung thixotroper Systeme mit geeigneter Fließgrenze, beliebig miteinander kombiniert werden können, um die Eigenschaften der Zubereitung weiter zu optimieren.

Die Zubereitung besteht aus einer formstabilen Lipid-/Emulgatormischung, die dispergiertes Wasser mit einer Tröpfchengröße unter 50 Mikrometer enthält

Anhand dieser erfindungsgemäßen Vorgaben kann der Fachmann ohne erfinderisch tätig zu werden eine erfindungsgemäße Zubereitung herstellen. Die Zubereitung ist somit durch die Eigenschaft charakterisiert, dass sie
- beim Verreiben und/oder Verteilen auf der Haut und/oder dem Haar schmilzt und/oder
- aufgrund von Scherkräften völlig oder teilweise flüssig wird und/oder
- sich mit den Lipiden des Hautsebums vermischt und/oder sich darin löst.

Erfindungsgemäß wird das Kosmetikum rückstandsfrei auf der Haut und/oder dem Haar aufgebracht. Rückstandfrei bedeutet hierbei, dass mit dem blossen Auge keinerlei Rückstände auf der haut oder dem Haar sichtbar bleiben.

Mehrere erfindungsgemäße Kugeln können gemeinsam in einer Packung aus Papier, Metall oder Plastik etc. oder einzeln oder zu mehreren durch weitere dünne Verpackungen ähnlich Bonbon-Papier oder in einer Blisterverpackung voneinander getrennt gelagert werden.

Vorteilhaft ist insbesondere die Kombination der erfindungsgemäßen Zubereitungen mit einer Blisterverpadcung, die während der Lagerung die einzelnen Kugeln voneinander trennt und so ein Zusammenfügen einzelner Kugeln verhindert. Dies kann auch durch Umwickeln der einzelnen Kugeln mit dünnen Folien aus Papier, Metall oder Plastik erreicht werden. Außerdem können die Zubereitungen in Röhrchen z.B aus Polystyrol verpackt werden, oder in Folien eingesiegelt werden. Neben Folien aus Zellglas, Aluminium und Papier können auch Kunststoff-Folien verwendet werden. Im Allgemeinen dient PE (Polymerisationsgrad von 3000-4000) als Material für derartige Packmittel. Weitere Möglichkeiten sind Durchdrückpackungen, bei denen auf eine Kunststoff-Folie beispielsweise eine Aluminiumfolie aufgesiegelt ist, oder Schrumpfpackungen. Die Zubereitungen können z.B auch in Faltschachteln, Kartons, Dosen, oder Kunststofftüten eingebracht werden.

Die Entnahme einzelner erfindungsgemäßer Kugeln kann durch einfaches Herausnehmen mit der Hand erfolgen. Die Kugel weisen erfindungsgemäß eine handhabbare Größe auf. Es ist aber auch möglich, die Entnahme der erfindungsgemäßen Zubereitung durch ein geeignetes Spendersystem zu erleichtern. Hierzu können z.B. durch Betätigen eines einfachen Mechanismus einzelne Kugeln aus dem Spendersystem freigegeben werden. Beispiel hierfür sind die von der Firma PEZ International AG unter der Marke "PEZ" vertriebenen Spendersysteme für Bonbons und andere Süßwaren.

Vorteilhaft sind aber auch Spendersysteme bei denen auf einer runden Scheibe die Kugeln schneckenförmig in Vertiefungen eingelagert sind, und durch einen Dosiermechanismus einzeln zu entnehmen sind. Besonders vorteilhaft sind hierbei Ausführungen, die von ihrer äußeren Form her an bekannte Kosmetikprodukte, beispielsweise die bekannte NIVEA-Dose, erinnern, da dies die Gefahr einer Verwechselung mit Lebensmitteln reduziert.

Bei der Anwendung werden einzelne Kugeln entnommen und auf der Haut verrieben. Das Produkt wird dabei durch Schmelzen, Scheren oder Lösen der festen Produktbestandteile niedriger viskos und gut verteilbar und löst sich auf bzw. in der Haut oder dem Haar auf. Dem Fachmann ist offensichtlich, dass die erfindungsgemäße Zubereitung durchaus feste Bestandteile enthalten können, deren Lösen während der Applikation weder möglich noch erwünscht ist, nämlich Feststoffe, wie sie bereits in herkömmlichen Kosmetika eingesetzt werden, ohne dass der Verbraucher bemerkt, dass sie in fester Form vorliegen. Beispiele hierfür sind Füllstoffe, Lichtschutz- und Farbpigmente.

Der Anwender entnimmt eine oder mehrere der Kugeln und verreibt sie auf der Haut, wie er es ansonsten mit Hautcreme aus der Dose oder Tube gewohnt ist. Vorteilhaft ist hier jedoch dass er eine vorportionierte Menge ohne Überreste und Verpackung anwenden kann.

Der Vorteil der erfindungsgemäßen Zubereitung ist die bequeme einfache Einmalanwendung für Zwischendurch. Ähnlich dem Schminken oder Einfetten der Lippen ist somit auch ein Eincremen oder die Hautpflege unterwegs möglich. Darüber hinaus kann der Verbraucher einzelne Kugeln auch anderen Verbrauchern anbieten. Dies ist bei herkömmlichen kosmetischen Produkten zwar auch möglich, aber das gemeinsame Benutzen beispielsweise einer Creme aus ein und demselben Tiegel gleicht psychologisch einer körperlichen Berührung. Deshalb existiert hier eine Hemmschwelle, die durch die vorliegende Erfindung beseitigt wird.

Es ist auch möglich erfindungsgemäße Kugeln mit unterschiedlichen Eigenschaften (z.B. Parfum, Farbe, Hautgefühl, Lichtschutzfaktor, enthaltene Wirkstoffe und Kombinationen dieser Eigenschaften) in einer Verpackung anzubieten, was bei herkömmlichen Hautpflegeprodukten nicht möglich ist.

Ein weiterer Vorteil ist, dass die Anwendung der erfindungsgemäßen Kugeln einigen Anwendern, vor allem Kindern, mehr Spaß macht als die Anwendung herkömmlicher kosmetischer Produkte. Dies kann es den Eltern erleichtern, ihre Kinder vor schädlichen Umwelteinflüssen wie beispielsweise UV-Strahiung zu schützen. Dem Fachmann ist bekannt, dass ein großes Problem der Schutz kosmetischer Produkte vor Pilzen, Hefen und Bakterien ist, die während der Anwendung in das Produkt gelangen. Dies geschieht vor allem durch das Anfassen des Produktes durch den Verbraucher während der Entnahme. Es ist offensichtlich, dass die erfindungsgemäßen Kugeln hier einen weiteren Vorteil bieten, da der Verbraucher nur diejenigen Kugeln berührt, die er auch sofort anwendet. Die übrigen Kugeln bleiben so vor mikrobiellem Befall geschützt, weswegen im Vergleich zu herkömmlichen kosmetischen Produkten mit geringeren Gehalten an Konservierungsmitteln gearbeitet werden kann. Da Konservierungsmittel zu den weniger verträglichen kosmetischen Rohstoffen gehören, ist so eine verbesserte Verträglichkeit der Produkte erreichbar, was einen weiteren Vorteil der vorliegenden Erfindung darstellt.

Die kugelförmige Zubereitung kann sich zusammensetzen aus Wachsen, Fetten, Ölen, Silikonölen, Wasser Glycerin und anderen Polyolen, sowie anderen wasserlöslichen oder öllöslichen Wirk-, Hilfs- oder Zusatzstoffen. Die Kugel hat ausreichende Schlagstabilität, um mechanische Belastungen während Herstellung und Lagerung auszuhalten, und ist dünn genug, sich bei der Applikation schnell auf der Haut verteilen zu lassen.

Die Lipidphase der Kugeln wird vorteilhaft aus Wachsen wie Ceresin, Ozokerit, Esterwachsen, Glyceridwachsen und/oder Fettalkoholen sowie festen Emulgatoren und deren Mischungen aufgebaut. Die Wachse können ihrer Herkunft nach Naturwachse, modifizierte Naturwachse, teilsynthetisch oden vollsynthetisch sein.
Alle Bestandteile werden so ausgewählt, dass sie die geforderte Form- und Temperaturstabilität gewährleisten, die Eintrocknung durch Verdunsten verhindern und bei der Applikation schnell schmelzen, aufgrund von Scherkräften völlig oder teilweise flüssig werden oder sich im Füllmaterial auflösen.
Zur Optimierung der elastischen Eigenschaften können Polymere in die Zubereitung eingearbeitet werden. Geeignete Polymere sind Celluloseether, Polyvinylpyrrolidon und seine Derivate, Polyacrylate oder Polymethacrylate, sowie Eudragit.
Die erfindungsgemäße Kugel setzt sich bevorzugt aus Wachsen zusammen, die aus der Gruppe der
- natürlichen Wachse, besonders bevorzugt Carnaubauwachs, Candelillawachs, Shellackwachs, Beerenwachs (Rhus Verniciflura), Shea Butter (Butyrospermum Parkii), hydrierte Pflanzenöle, wie hydriertes Palm- oder Rapsöl, Bienenwachs, Wollwachs (Eucerit)
- Mono-, Di - und Triglyceride aus höheren gesättigten Fettsäuren mit 10 - 30 Kohlenstoffatomen oder Mischungen hieraus, besonders bevorzugt Glyceryltripalmitat (Dynasan 116) und/oder Glycerylstearat, Glyceryltribehenat (Syncrowax HRC)
- höheren gesättigten Fettalkohole, besonders bevorzugt solche mit 14 - 30 Kohlenstoffatomen, ganz besonders bevorzugt Stearylalkohol und/oder Behenylalkohol und/oder Cetylalkohol
- synthetischen Ester, bevorzugt C16-36 Alkylhydroxystearoylstearat, Stearylstearat, Cetearylbehenat, C20-40 Alkylstearat, besonders bevorzugt Cetylpalmitat, Methylpalmitat, Myristylmyristat, Cetylrhicinoleat
- Polymerwachse, bevorzugt Polyethylen, Polypropylen, Polyvinylether, Polydecen besonders bevorzugt Polyvinylstearylether und hydriertes Polydecen,
- Copolymere, besonders bevorzugt solche aus Ethylen- und Vinylacetat sowie aus Polyvinylpyrrolidon und Hexadecen,
- Kohlenwasserstoffe/ Paraffinwachse, besonders bevorzugt Cera Microcristallina, Paraffinwachs, Ceresin, Ozokerit
- Silikonwachse
- chemisch modifizierten Wachse
- beliebigen Mischungen aus Wachsen der genannten Gruppen gewählt werden.

Erfindungsgemäß besonders bevorzugte Wachse zur Herstellung der erfindungsgemäßen Zubereitung sind Cetylpalmitat, Cetylrhicinoleat, Bienenwachs, hydrierte Kokosglyceride, Methylpalmitat, Candelillawachs, Carnaubawachs, Paraffinwachs, Ceresin, Ozokerit, Myristylmyristat, Tripalmitin, Tribehenin, Glycerylpalmitostearat, hydriertes Rapsöl und C15-C40 Alkylstearylstearat.

Die erfindungsgemäßen Kugeln können die üblichen Hilfs- und Zusatzstoffe enthalten, die dem Fachmanne natürlich bekannt sind und wie sie üblicherweise in Kosmetika verwendet werden, z.B. Konservieungsmittel, Bakterizide, desodorierend wirkende Substanzen, Antitranspirantien, Insektenrepellentien, Vitamine, Mittel zum Verhindern des Schäumens, Farbstoffe, Pigmente mit färbender Wirkung, Geschmacksstoffe, Vergällungsmittel, Parfums, Verdickungsmittel, weichmachende Substanzen, anfeuchtende und/oder feuchthaltende Substanzen, Antioxidantien, UV-Filtersubstanzen, Sensorikadditive, Filmbildner, Tenside, Emulgatoren, Fette, Öle, Wachse, Wirkstoffe oder andere übliche Bestandteile einer kosmetischen Formulierung wie Alkohole, Polyole, stabilisierende Polymere, Schaumstabilisatoren, Elektrolyte, organische Lösungsmittel oder Silikonderivate.

Als vorteilhafte W/O-Emulgatoren können eingesetzt werden: Fettalkohole mit 8 bis 30 Kohlenstoffatomen, Monoglycerinester gesättigter und/oder ungesättigter, verzweigter oder unverzweigter Alkancarbonsäuren einer Kettenlänge von 8 bis 24, insbesondere 12 bis 20 Kohlenstoffatomen, Diglycerinester gesättigter und/oder ungesättigter, verzweigter oder unverzweigter Alkancarbonsäuren einer Kettenlänge von 8 bis 24, insbesondere 12 bis 20 Kohlenstoffatomen, Monoglycerinether gesättigter und/oder ungesättigter, verzweigter oder unverzweigter Alkohole einer Kettenlänge von 8 bis 24, insbesondere 12 bis 20 Kohlenstoffatomen, Diglycerinether gesättigter und/oder ungesättigter, verzweigter oder unverzweigter Alkohole einer Kettenlänge von 8 bis 24, insbesondere 12 bis 20 Kohlenstoffatomen, Polypropylenglykolester gesättigter und/oder ungesättigter, verzweigter oder unverzweigter Alkancarbonsäuren einer Kettenlänge von 8 bis 24, insbesondere 12 bis 20 Kohlenstoffatomen, Polyglycerylester gesättigter und/oder ungesättigter, verzweigter oder unverzweigter Alkancarbonsäuren einer Kettenlänge von 8 bis 24, Insbesondere 12 bis 20 Kohlenstoffatomen, Sorbitanester gesättigter und/oder ungesättigter, verzweigter oder unverzweigter Alkancarbonsäuren einer Kettenlänge von 8 bis 24, insbesondere 12 bis 20 Kohlenstoffatomen Lanolinalkohol

Bevorzugte W/O-Emulgatoren sind Polyglyceryl-3 Diisostearat, Polyglyceryl-4 Isostearat, Polyglyceryl-2 Dipolyhydroxystearat, Cetyl PEG/PPG-10-1- Dimethicone, PEG-30 Dipolyhydroxystearat, PEG-40 Sorbitanperisostearat, Cetyldimethiconecopolyol, PEG-7 Hydrogenated Castor Oil, PEG 45/Dodecylglycolcopolymer, PEG 22/ Dodecylglycolgopolymer, Pentaerythritylisostearat, Isostearyldiglycerylsuccinat, Sorbitanisostearat, Polyglyceryl-2 Sesquiisostearat, Glycerylisostearat, Sorbitanstearat, Glycerylstearat, PEG-25 Hydrogenated Castor Oil, PEG-40 Sorbitanperoleat, Sorbitanoleat, PEG-40 Sorbitanperisostearat, Polyglyceryl-3 Oleat, Polyglyceryl-2 Sesquioleat und Polyglyceryl-4 Isostearat.

Besonders bevorzugte W/O-Emulgatoren sind Poylethylenglycol-45/Dodecylglycolcopolymer, Polyglyceryl-3-Diisostearat, PEG-30 Dipolyhydroxystearate, Sorbitanisostearate, Sorbitan Stearate, Glyceryl Isostearate, Glyceryl Stearate und Sorbitah Oleate.

Es ist auch möglich, auf das Herabsetzen der Grenzflächenenergie durch Emulgatoren oder Tenside zu verzichten und statt dessen die Grenzfläche durch Anlagerung von in beiden Phasen unlöslichen Partikeln zu stabilisieren. Hierzu können natürliche oder künstliche Polymere (Polyethylen, Nylon, Stärke und ihre Derivate) oder anorganische Partikel (TiO₂, Al₂O₃, BaSO₄, BN, Silikate, Alumosilikate, SiO₂, Aerosil) verwendet werden.

Die Ölphase, die Lipide, der erfindungsgemäßen Formulierungen wird vorteilhaft gewählt aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Sojaöl, Erdnussöl, Rapsöl,' Mandelöl, Palmöl, Kokosöl, Rizinusöl, Weizenkeimöl, Traubenkernöl, Distelöl, Nachtkerzenöl, Macadamianussöl und dergleichen mehr.

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Octylpalmitat, Octylcocoat, Octylisostearat, Octyldodeceylmyristat, Octyldodekanol, Cetearylisononanoat, Isopropylmyristat, Isopropylpalmitat, Isopropylstearat, lsopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl.

Ferner kann die Ölphase vorteilhaft gewählt werden aus der Gruppe der Dialkylether und Dialkylcarbonate, vorteilhaft sind z. B. Dicaprylylether (*Cetiol OE*) und/oder Dicaprylylcarbonat, beispielsweise das unter der Handelsbezeichnung *Cetiol CC* erhältlich ist.

Es ist ferner bevorzugt, die Ölkomponenten aus der Gruppe Isoeikosan, Neopentylglykoldiheptanoat, Propylenglykoldicaprylat/dicaprat, Caprylic/Capric/Triglyceride, Caprylic/Capric/Diglycerylsuccinat, Butylenglykol Dicaprylat/Dicaprat, C₁₂₋₁₃-Alkyllactat, Di-C₁₂₋₁₃-Alkyltartrat, Triisostearin, Dipentaerythrityl Hexacaprylat/Hexacaprat, Propylenglykolmonoisostearat, Tricaprylin, Dimethylisosorbid zu wählen. Es ist insbesondere vorteilhaft, wenn die Ölphase der erfindungsgemäßen Formulierungen einen Gehalt an C₁₂₋₁₅-Alkylbenzoat aufweist oder vollständig aus diesem besteht.

Vorteilhafte Ölkomponenten sind ferner z. B. Butyloctylsalicylat (beispielsweise das unter der Handelsbezeichnung *Hallbrite BHB* erhältliche), Hexadecylbenzoat und Butyloctylbenzoat und Gemische davon (*Hallstar AB*) und/oder Diethylhexylnaphthalat (*Hallbrite TQ oder Corapan TQ von H&R*).

Auch beliebige Abmischungen solcher Öl- und Wachskomponenten sind vorteilhaft im Sinne der vorliegenden Erfindung einzusetzen.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene und Isohexadecan. Unter den Polyolefinen sind Polydecene die bevorzugten Substanzen.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Silikonöle sind hochmolekulare synthetische polymere Verbindungen, in denen Silicium-Atome über Sauerstoff-Atome ketten- und/oder netzartig verknüpft und die restlichen Valenzen des Siliciums durch Kohlenwasserstoff-Reste (meist Methyl-, seltener Ethyl-, Propyl-, Phenyl-Gruppen u. a.) abgesättigt sind. Systematisch werden die Silikonöle als Polyorganosiloxane bezeichnet. Die methylsubstituierten Polyorganosiloxane, welche die mengenmäßig bedeutendsten Verbindungen dieser Gruppe darstellen und sich durch die folgende Strukturformel auszeichnen 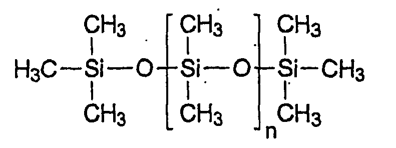 werden auch als Polydimethylsiloxan bzw. Dimethicon (INCI) bezeichnet. Dimethicone gibt es in verschiedenen Kettenlängen bzw, mit verschiedenen Molekulargewichten.

Besonders vorteilhafte Polyorganosiloxpne im Sinne der vorliegenden Erfindung sind beispielsweise Dimethylpolysiloxane [Poly(dimethylsiloxan)], welche beispielsweise unter den Handelsbezeichnungen Abil 10 bis 10 000 bei Th. Goldschmidt erhältlich sind. Ferner vorteilhaft sind Phenylmethylpolysiloxane (INCI: Phenyl Dimethicone, Phenyl Trimethicone), cyclische Silikone (Octamethylcyclotetrasiloxan bzw. Decamethylcyclopentasiloxan), welche nach INCI auch als Cyclomethicone bezeichnet werden, aminomodifizierte Silikone (INCI: Amodimethicone) und Silikonwachse, z. B. Polysiloxan-Polyalkylen-Copolymere (INCI: Stearyl Dimethicone und Cetyl Dimethicone) und Dialkoxydimethylpolysiloxane (Stearoxy Dimethicone und Behenoxy Stearyl Dimethicone), welche als verschiedene Abil-Wax-Typen bei Th. Goldschmidt erhältlich sind. Aber auch andere Silikonöle sind vorteilhaft im Sinne der vorliegenden Erfindung zu verwenden, beispielsweise Cetyldimethicon, Hexamethylcyclotrisiloxan, Polydimethylsiloxan, Poly(methylphenylsiloxan).

Es ist ebenfalls vorteilhaft, den Zubereitungen im Sinne der vorliegenden Erfindung übliche Antioxidantien zuzufügen. Erfindungsgemäß können als günstige Antioxidantien alle für kosmetische und/oder dermatologische Anwendungen geeigneten oder gebräuchlichen Antioxidantien verwendet werden.
Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Camosin, L-Camosin und deren Derivate (z.B. Anserin), Carotinoide, Carotine (z.B. α-Carotin, β-Carotin, Lycopin) und deren Derivate, Retinoide wie zum Beispiel Retinol, Retinal und/oder Retinsäure und die jeweiligen Ester, α-Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl-und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Zitronensäure, Milchsäure, Äpfelsäure), Huminsäure, Gallensäure, Gallenextrakte, Bilirubin, Biliverdin, EDTA, IDS, EGTA und deren Derivate, ungesättigte Fettsäuren und deren Derivate (z.B. γ-Linolensäure, Linolsäure, Ölsäure), Folsäure und deren Derivate, -2-Aminopropionsäurediessigsäure, Flavonoide, Polyphenole, alpha-Glycosylrutin, Propylgallat, Catechine, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), sowie Koniferylbenzoat des Benzolëharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.
Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 0,1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Weiterhin können der erfindungsgemäßen Zubereitung UV-Filtersubstanzen zugesetzt werden. Es ist damit bevorzugt die erfindungsgemäßen Zubereitungen als Lichtschutzformulierungen anzuwenden.

Besonders vorteilhafte bei Raumtemperatur flüssige UV-Filtersubstanzen im Sinne des vorliegenden Erfindung sind Homomenthylsalicylat (INCI: Homosalate), 2-Ethylhexyl-2-cyano-3,3-diphenylacrylat (INCI: Octocrylene), 2-Ethylhexyl-2-hydroxybenzoat (2-Ethylhexylsalicylat, Octylsalicylat, INCI: Octyl Salicylate) und Ester der Zimtsäure, vorzugsweise 4-Methoxyzimtsäure (2-ethylhexyl)ester (2-Ethylhexyl-4-methoxycinnamat, INCI: Octyl Methoxycinnamate) und 4-Methoxyzimtsäureisopentylester (Isopentyl-4-methoxy-cinnamat, INCI: Isoamyl p-Methoxycinnamate).
Bevorzugte anorganische Pigmente sind Metalloxide und/oder andere in Wasser schwerlösliche oder unlösliche Metallverbindungen, insbesondere Oxide des Titans (TiO₂), Zinks (ZnO), Eisens (z. B: Fe₂O₃), Zirkoniums (ZrO₂), Siliciums (SiO₂), Mangans (z. B. MnO), Aluminiums (Al₂O₃), Cers (z. B. Ce₂O₃), Mischoxide der entsprechenden Metalle sowie Abmischungen aus solchen Oxiden sowie das Sulfat des Bariums (BaSO₄).
Die Pigmente können vorteilhaft im Sinne der vorliegenden Erfindung auch in Form kommerziell erhältlicher öliger oder wässriger Vordispersionen zur Anwendung kommen. Diesen Vordispersionen können vorteilhaft Dispergierhilfsmittel und/oder Solubilisationsvermittler zugesetzt sein.
Die Pigmente können erfindungsgemäß vorteilhaft oberflächlichbehandelt ("gecoatet") sein, wobei beispielsweise ein hydrophiler, amphiphiler oder hydrophober Charakter gebildet werden bzw. erhalten bleiben soll. Diese Oberflächenbehandlung kann darin bestehen, dass die Pigmente nach an sich bekannten Verfahren mit einer dünnen hydrophilen und/oder hydrophoben anorganischen und/oder organischen Schicht versehen werden. Die verschiedenen Oberflächenbeschichtungen können im Sinne der vorliegenden Erfindung auch Wasser enthalten.
Anorganische Oberflächenbeschichtungen im Sinne der vorliegenden Erfindung können bestehen aus Aluminiumoxid: (Al₂O₃), Aluminiumhydroxid Al(OH)₃, bzw. Aluminiumoxidhydrat (auch: Alumina, CAS-Nr.: 1333-84-2), Natriumhexametaphosphat (NaPO₃)₆, Natriummetaphosphat (NaPO₃)ₙ, Siliciumdioxid (SiO₂) (auch: Silica, CAS-Nr.: 7631-86-9), oder Eisenoxid (Fe₂O₃). Diese anorganischen Oberflächenbeschichtungen können allein, in Kombination und/oder in Kombination mit organischen Beschichtungsmaterialien vorkommen.
Organische Oberflächenbeschichtungen im Sinne der vorliegenden Erfindung können bestehen aus pflanzlichem oder tierischem Aluminiumstearat, pflanzlicher oder tierischer Stearinsäure, Laurinsäure, Dimethylpolysiloxan (auch: Dimethicone), Methylpolysiloxan (Methicone), Simethicone (einem Gemisch aus Dimethylpolysiloxan mit einer durchschnittlichen Kettenlänge von 200 bis 350 Dimethylsiloxan-Einheiten und Silicagel) oder Alginsäure. Diese organischen Oberflächenbeschichtungen können allein, in Kombination und/oder in Kombination mit anorganischen Beschichtungsmaterialien vorkommen.
Erfindungsgemäß geeignete Zinkoxidpartikel und Vordispersionen von Zinkoxidpartikeln sind unter folgenden Handelsbezeichnungen bei den aufgeführten Firmen erhältlich:

| Handelsname | Coating |
|---|---|
| Z- Cote HP1 | 2% Dimethicone |
| Z- Cote | / |
| ZnO NDM | 5% Dimethicone |

Geeignete Titandioxidpartikel und Vordispersionen von Titandioxidpartikeln sind unter folgenden Handelsbezeichnungen bei den aufgeführten Firmen erhältlich:

| Handelsnamen | Coating |
|---|---|
| MT-100TV | Aluminiumhydroxid / Stearinsäure |
| MT-100Z | Aluminiumhydroxid / Stearinsäure |
| Eusolex T-2000 | Alumina / Simethicone |
| Titandioxid T805 (Uvinul TiO₂) | Octyltrimethylsilan |

Vorteilhafte UV-A-Filtersubstanzen im Sinne der vorliegenden Erfindung sind Dibenzoylmethanderivate, insbesondere das 4-(tert.-Butyl)-4'-methoxydibenzoylmethan (CAS-Nr. 70356-09-1), welches von Givaudan unter der Marke Parsol^{®} 1789 und unter der Handelsbezeichnung Eusolex® 9020 verkauft wird.

Ebenfalls geeignete UV-A Filtersubstanzen sind Hydroxybenzophenone. Diese zeichnen sich durch die folgende Strukturformel aus: 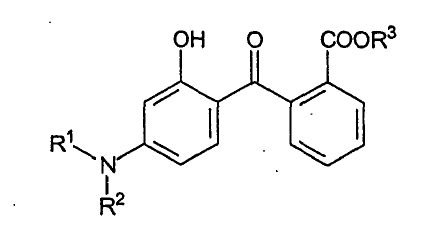 worin
- R¹ und R² unabhängig voneinander Wasserstoff, C₁-C₂₀-Alkyl, C₃-C₁₀-Cycloalkyl oder C₃-C₁₀-Cycloalkenyl bedeuten, wobei die Substituenten R¹ und R² gemeinsam mit dem Stickstoffatom, an das sie gebunden sind, einen 5- oder 6-Ring bilden können und
- R³ einen C₁-C₂₀-Alkyl Rest bedeutet.

Ein besonders vorteilhaftes Hydroxybenzophenon im Sinne der vorliegenden Erfindung ist der 2-(4'-Diethylamino-2'-hydoxybenzoyl)-benzoesäurehexylester (auch: Aminobenzophenon), welcher sich durch folgende Struktur auszeichnet: 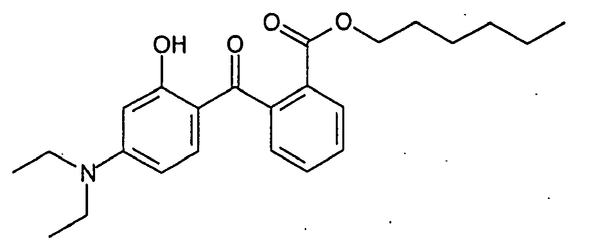 und unter dem Uvinul A Plus bei der Fa. BASF erhältlich ist.
Die Gesamtmenge an einem oder mehreren Hydroxybenzophenonen in den fertigen kosmetischen öder dermatologischen Zubereitungen wird vorteilhaft aus dem Bereich 0,01 Gew.-% bis 20 Gew.-%, vorzugsweise von 0,1 bis 10 Gew.-% gewählt, jeweils bezogen auf das Gesamtgewicht der Zubereitungen.
Vorteilhafte weitere UV-Filtersubstanzen im Sinne der vorliegenden Erfindung sind sulfonierte, wasserlösliche UV-Filter, wie z. B.:
- Phenylen-1,4-bis-(2-benzimidazyl)-3,3'-5,5'-tetrasulfonsäure und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz mit der INCI-Bezeichnung Bisimidazylate (CAS-Nr.: 180898-37-7), welches beispielsweise unter der Handelsbezeichnung Neo' Heliopan AP erhältlich ist;
- Salze der 2-Phenylbenzimidazol-5-sulfonsäure, wie ihr Natrium-, Kalium- oder ihr Triethanolammonium-Salz sowie die Sulfonsäure selbst mit der INCI Bezeichnung Phenylbenzimidazole Sulfonsäure (CAS.-Nr. 27503-81-7), welches beispielsweise unter der Handelsbezeichnung Eusolex 232 oder unter Neo Heliopan Hydrö erhältlich ist;
- 1,4-di(2-oxo-10-Sulfo-3-bornylidenmethyl)-Benzol (auch: 3,3'-(1,4-Phenylendimethylene)-bis-(7,7-dimethyl-2-oxo-bicyclo-[2.2.1]hept-1-ylmethan Sulfonsäure) und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird. Benzol-1,4-di(2-oxo-3-bornylidenmethyl-10-sulfonsäure) hat die INCI-Bezeichnung Terephtalidene Dicampher Sulfonsäure (CAS.-Nr.: 90457-82-2) und ist beispielsweise unter dem Handelsnamen Mexoryl SX erhältlich;
- Sulfonsäure-Derivate des 3-Benzylidencamphers, wie z. B. 4-(2-Oxo-3-bornylidenmethyl)benzolsulfonsäure, 2-Methyl-5-(2-oxo-3-bornylidenmethyl)sulfonsäure und deren Salze.

Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.
Vorteilhafte Breitbandfilter oder UV-B-Filtersubstanzen sind beispielsweise Triazinderivate, wie z. B.
- 2,4-Bis-{[4-(2-Ethyl-hexyloxy)-2-hydroxy]-phenyl}-6-(4-methoxyphenyl)-1,3,5-triazin (INCI: Aniso Triazin), welches unter der Handelsbezeichnung Tinosorb® S erhältlich ist;
- Diethylhexylbutylamidotriazon (INCI: Diethylhexylbutamidotriazone), welches unter der Handelsbezeichnung UVASORB HEB erhältlich ist;
- 4,4',4"-(1,3,5-Triazin-2,4,6-triyltriimino)-tris-benzoësäure-tris(2-ethylhexylester), auch: 2,4,6-Tris-[anilino-(p-carbo-2'-ethyl-1'-hexyloxy)]-1,3,5-triazin (INCI: Ethylhexyl Triazone), welches unter der Warenbezeichnung UVINUL® T 150 vertrieben wird.

Ein vorteilhafter Breitbandfilter im Sinne der vorliegenden Erfindung ist auch das 2,2'-Methylen-bis-(6-(2H-benzotriazol-2-yl)-4-(1,1,3,3-tetramethylbutyl)-phenol), welches unter der Handelsbezeichnung Tinosorb® M erhältlich ist.
Vorteilhafter Breitbandfilter im Sinne der vorliegenden Erfindung ist ferner das 2-(2H-benzotriazol-2-yl)-4-methyl-6-[2-methyl-3-[1,3,3,3-tetramethyl-1-[(trimethylsilyl)oxy]disiloxanyl]propyl]-phenol (CAS-Nr.: 155633-54-8) mit der INCI-Bezeichnung Drometrizole Trisiloxahe, welches unter der Handelsbezeichnung Mexoryl® XL erhältlich ist.

Die weiteren UV-Filtersubstanzen können öllöslich oder wasserlöslich sein.

Vorteilhafte öllösliche UV-B- und/oder Breitband-Filtersubstanzen im Sinne der vorliegenden Erfindung sind z. B.:
■ 3-Benzylidencampher-Derivate, vorzugsweise 3-(4-Methylbenzyliden)campher, 3-Benzylidencampher;
■ 4-Aminobenzoesäure-Derivate, vorzugsweise 4-(Dimethylamino)-benzoesäure(2-ethylhexyl)ester, 4-(Dimethylamino)benzoesäureamylester;
■ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon
■ sowie an Polymere gebundene UV-Filter.
■ 3-(4-(2,2-bis Ethoxycarbonylvinyl)-phenoxy)propenyl)-methoxysiloxan/Dimethylsiloxan - Copolymer welches beispielsweise unter der Handelsbezeichnung Parsol® SLX erhältlich ist.

Vorteilhafte wasserlösliche Filtersubstanzen sind z. B.:
Sulfonsäure-Derivate des 3-Benzylidencamphers, wie z. B. 4-(2-Oxo-3-bornylidenmethyl)benzolsulfonsäure, 2-Methyl-5-(2-oxo-3-bornylidenmethyl)sulfonsäure und deren Salze.
Eine weiterere erfindungsgemäß vorteilhaft zu verwendende Lichtschutzfltersubstanz ist das Ethylhexyl-2-cyano-3,3-diphenylacrylat (Octocrylen), welches unter der Bezeichnung Uvinul^{®} N 539 erhältlich ist.
Besonders vorteilhafte Zubereitungen im Sinne der vorliegenden Erfindung, die sich durch einen hohen bzw. sehr hohen UV-A- und/oder UV-B-Schutz auszeichnen, enthalten neben der oder den erfindungsgemäßen Filtersubstanz(en) bevorzugt ferner weitere UV-A- und/oder Breitbandfilter, insbesondere Dibenzoylmethanderivate [beispielsweise das 4-(tert.-Butyl)-4'-methoxydibenzoylmethan], Phenylen-1,4-bis-(2-benzimidazyl)-3,3'-5,5'-tetrasulfonsäure und/oder ihre Salze, das 1,4-di(2-oxo-10-Sulfo-3-bornylidenmethyl)-Benzol und/oder dessen Salze und/oder das 2,4-Bis-{[4-(2-Ethylhexyloxy)-2-hydroxy]-phenyl}-6-(4-methoxyphenyl)-1,3,5-triazin, jeweils einzeln oder in beliebigen Kombinationen miteinander.
Die Liste der genannten UV-Filter, die im Sinne der vorliegenden Erfindung eingesetzt werden können, soll selbstverständlich nicht limitierend sein.
Vorteilhaft enthalten die erfindungsgemäßen Zubereitungen die Substanzen, die UV-Strahlung im UV-A- und/oder UV-B-Bereich absorbieren, in einer Gesamtmenge von z. B. 0,1 Gew.-% bis 30 Gew.-%, vorzugsweise 0,5 bis 20 Gew.-%, insbesondere 1,0 bis 15,0 Gew.-%, jeweils bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar dienen.

Erfindungsgemäße als Emulsionen vorliegende Zubereitungen enthalten insbesondere vorteilhaft ein oder mehrere Hydrocolloide. Diese Hydrocolloide können vorteilhaft gewählt werden aus der Gruppe der Gummen, Polysaccharide, Cellulosederivate, Silica und Aerosile, Schichtsilikate, Polyacrylate und/oder anderen Polymeren.

Zu den Gummen zählt man Pflanzen- oder Baumsäfte, die an der Luft erhärten und Harze bilden oder Extrakte aus Wasserpflanzen. Aus dieser Gruppe können vorteilhaft im Sinne der vorliegenden Erfindung gewählt werden beispielsweise Gummi Arabicum, Johannisbrotmehl, Tragacanth, Karaya, Guar Gummi, Pektin, Gellan Gummi, Carrageen, Agar, Algine, Chondrus, Xanthan Gummi.

Weiterhin vorteilhaft ist die Verwendung von derivatisierten Gummen wie z.B. Hydroxypropyl Guar (Jaguar® HP 8).

Unter den Polysacchariden und -derivaten befinden sich z.B. Hyaluronsäure, Chitin und Chitosan, Chondroitinsulfate, Stärke und Stärkederivate.

Unter den Cellulosederivaten befinden sich z.B. Methylcellulose, Carboxymethylcellulose, Hydroxyethylcellulose, Hydroxypropylmethylcellulose.

Unter den Schichtsilikaten befinden sich natürlich vorkommende und synthetische Tonerden wie z.B. Montmorillonit, Bentonit, Hektorit, Laponit, Magnesiumaluminiumsilikate wie Veegum®. Diese können als solche oder in modifizierter Form verwendet werden wie z.B. Stearylalkonium Hektorite.

Weiterhin können vorteilhaft auch Kieselsäuregele verwendet werden, zur deren Herstellung beispielsweise pyrogene Kieselsäure (beispielsweise die Aerosil-Tpyen 200, 300, 800, R 812 oder R 972) verwendet werden können

Vorteilhaft sind außerdem Taurate, z.B. Ammonium Acryloyldimethyltaurat / VP Copolymer.

Unter den Polyacrylaten befinden sich z.B. Carbopol Typen (Carbopol 980, 981, 1382, 5984, 2984, ETD 2001, ETD 2020, ETD 2050, Ultrez-10 oder Pemulen TR1 & TR2).

Unter den Polymeren befinden sich z.B. Polyacrylamide (Seppigel 305), Polyvinylalkohole, PVP, PVP / VA Copolymere, Polyglycole.

Die Wasserphase der Zubereitungen gemäß der vorliegenden Erfindung kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, Glycerin, Butylenglykol, Ethylenglykol, Ethylenglykolmonoethyl- oder -monobutylether, Propylenglykolmonomethyl, -monoethyl- oder -monobutylether, Diethylenglykolmonomethyloder -monoethylether, Ethylhexyloglycerin, Metpylpropandiol und analoge Produkte, Polymere, Schaumstabilisatoren, Elektrolyte wie beispielsweise Natriumchlorid oder Magnesiumsulfat sowie insbesondere ein oder mehrere Verdickungsmittel, welches oder welche vorteilhaft gewählt werden können aus der Gruppe Siliziumdioxid, Alumosilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, ETD 2020, ETD 2050, Ultrez 10, jeweils einzeln oder in Kombination.

Die Zubereitungen gemäß der vorliegenden Erfindung können ferner vorteilhaft auch Selbstbräunungssubstanzen enthalten, wie beispielsweise Dihydroxyaceton und/oder Melaninderivate in Konzentrationen von 0,1 Gew.-% bis zu 10 Gew.-%, bezogen auf das Gesamtgewicht der Zubereitung.

Ferner vorteilhaft können die Zubereitungen gemäß der vorliegenden Erfindung auch Repellentien zum Schutz vor Mücken, Zecken und Spinnen und dergleichen enthalten. Vorteilhaft sind z. B. N,N-Diethyl-3-methylbenzamid (Handelsbezeichnung: Metadelphene, "DEET"), Dimethylphtalat (Handelsbezeichnung: Palatinol M, DMP) sowie insbesondere 3-(N-n-Butyl-N-acetyl-amino)-propionsäureethylester (unter dem Handelsnamen Insekt Repellent® 3535 erhältlich). Die Repellentien können sowohl einzeln als auch in Kombination eingesetzt werden. Gleichfalls können aromatische Öle oder dergleichen eingesetzt werden.

Als Feuchthaltemittel und/oder hautbefeuchtende Stoffe werden Stoffe oder Stoffgemische bezeichnet, welche kosmetischen oder dermatologischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen. Vorteilhafte Moisturizer im Sinne der vorliegenden Erfindung sind beispielsweise Glycerin, Milchsäure und/oder Lactate, insbesondere Natriumlactat, Butylenglykol, Propylenglykol, Panthenol, Fucogel, Glycine Soja, Ethylhexyloxyglycerin, Pyrrolidoncarbonsäure und ihre Derivate sowie Harnstoff. Ferner ist es insbesondere von Vorteil, polymere Moisturizer aus der Gruppe der wasserlöslichen und/ oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 erhältlich ist. Moisturizer können vorteilhaft auch als Antifaltenwirkstoffe zur Prophylaxe und Behandlung kosmetischer oder dermatologischer Hautveränderungen, wie sie z. B. bei der Hautalterung auftreten, verwendet werden.

Die erfindungsgemäßen kosmetischen oder dermatologischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke-Octenylsuccinat und dergleichen), Pigmente, die weder hauptsächlich UV-Filter- noch färbende Wirkung haben (wie z. B. Bornitrid etc.) und/oder Aerosile^{®} (CAS-Nr. 7631-86-9).

Zur Anwendung werden die kugelförmigen Zubereitungen erfindungsgemäß in der für Kosmetika üblichen Weise auf die Haut und/oder die Haare aufgebracht und verrieben bzw. verteilt.

Die erfindungsgemäßen kosmetischen und/oder dermatologischen Zubereitungen können neben den erfindungsgemäßen Bestandteilen wie übliche kosmetische und/oder dermatologische Zubereitungen zusammengesetzt sein und dem kosmetischen und/oder dermatologischen Lichtschutz, zur Änderung oder Beeinflussung bestimmter Hautzustände, ferner zur Behandlung, der Pflege der Haut und/oder der Haare und als Schminkprodukt in der dekorativen Kosmetik dienen.

Entsprechend können kosmetische und/oder topische dermatologische Zusammensetzungen im Sinne der vorliegenden Erfindung, je nach ihrem Aufbau, beispielsweise verwendet werden als Hautpflegeprodukt, Hautschutzprodukt, Sonnenschutzprodukt, Haarkur, für die Tages- oder Nachtpflege, die Pflege bestimmter Hautareale wie Hände, Gesicht, Füße usw.

Auch ist die Verwendung der erfindungsgemäßen kugelförmigen kosmetischen Zubereitungen zur Prophylaxe und Behandlung der Symptome von Altershaut, zur Verhinderung und Verminderung der Entstehung und Ausbreitung von Fältchen und Falten sowie zur Behandlung und Pflege gealterter Haut erfindungsgemäß. So lässt sich vorteilhaft eine einzelne Kugel, enthaltend Ubichinon, Ubichinol, Retinol und Derivate, Dehydroepiandrosteron (DHEA), Isoflavonoide (insbes. Genistein, Daidzein), Creatin, Phytoöstrogene, Östrogen, Östradiol und Derivate, Niacinamid, Polyphenole (alpha-Glucosylrutin) oder eine andere, gegen Falten wirksame Substanz auf die Gesichtshaut auftragen und verteilen.

Weiterhin ist die Verwendung der erfindungsgemäßen kugelförmigen kosmetischen Zubereitungen zur Prophylaxe und Behandlung der Symptome trockener Haut bevorzugt. Geeignete Wirkstoffe für diesen Einsatzzweck sind: natürliche Öle (Sonnenblumen-, Nachtkerzensamen-, Jojoba-, Macadamiaöl, Rhizinusöl), Ceramide, insbesondere Ceramid I, III und VI, Cholesterin, Phytosterole, Fettsäuren mit einer Kettenlänge von C16-26, insbesondere Linolsäure, Carnitin und seine Derivate, Harnstoff, Polyole wie Glycerin, Butylenglykol, Propylenglykol und Dipropylenglykol, Pseudoceramide; Elektrolyte wie Natriumchlorid und Taurin, Fettalkohole sowie Wachse.

Außerdem ist die Verwendung der erfindungsgemäßen kugelförmigen kosmetischen Zubereitungen zur Prophylaxe und Behandlung der Symptome der sensiblen bzw. entzündeten Haut vorteilhaft. Bevorzugte Wirkstoffe für diesen Einsatzzweck sind: Inhaltsstoffe der Mariendistel, insbesondere Silymarin, Hamamelisextrakt, Kamillenextrakt, Inhaltsstoffe der Süßholzpflanze (Glycerrhizinsäure), Acetylsalicylsäure, Diclofenac, Pentacyclische Triterpene (Sericoside, Ursolsäure), Licochalcone und Panthenol.

Außerdem ist die Verwendung der erfindungsgemäßen kugelförmigen kosmetischen Zubereitungen zur Prophylaxe und Behandlung der Symptome der fehiplgmentierten Haut vorteilhaft. Bevorzugte Wirkstoffe für diesen Einsatzzweck sind: Tyrosinaseinhibitoren, Hydrochinonderivate, Dioic Acid, Liponsäure und ihre Derivate sowie Kojisäure.

Es ist gegebenenfalls möglich und vorteilhaft, die erfindungsgemäßen Zusammensetzungen als Grundlage für pharmazeutische Formulierungen zu verwenden. Weiterhin ist die Verwendung der erfindungsgemäßen kugelförmigen kosmetischen Zubereitungen zur Prophylaxe und Behandlung der Symptome kranker Haut bevorzugt. Relevante aber nicht ausschließliche krankhafte Hautzustände sind Psoriasis, Akne, Neurodermitis und andere atopische Erkrankungen wie atopische Dermatitis, Hautkrebs, Herpes, Mykosen, Ichthyosis, Pityriasis, Seborrhöe, Pellagra, Kontaktekzeme und Allergien. Geeignete Wirkstoffe für solche Einsatzzwecke sind Antibiotika wie Fusidinsäure, Erythromycin, Sulfadiazin, Clindamycin, Tetracycline, Tyrothricin Aminoglycoside, Bacitracin, Chloramphenicol, Virustatika (z.B. Aciclovir, Idoxuridin, Penciclovir), Antimykotika (z.B. Nystatin, Amphotericin, Clotrimazol, Econazol, Ketoconazol, Naftifin, Terbinafin), Allethrin, Zytostatika (5-Fluorouracil), Antiphlogistica (Hydrocortison, Bethamethason; Prednisolon, Triamcinolonacetonid, Dexamethason, Diclofenac, Bufexamac), Immunosuppressiva (Cyclosporin A, Interferon-beta), Antipsoriatica (Dithranol, Psoralen, Tazaroten), Aknemittel (Retinsäure, Isotretinoin, Benzoylperoxid, Adapalen); Capsaicin, Azelainsäure, Keratolytika (Salicylsäure, Milchsäure), Antihistaminika (Azelastin, Levocabastin, Dinatrium-cromoglycin); Antipsoriatika (Dithranol, Calcitriol, Psoralen) und Vitamine (besonders die A-, B- und C-Vitamine).

Eine mögliche und erfindungsgemäße, vorteilhafte Anwendung ist es, Kugeln mit unterschiedlichen Verwendungszwecken in einer Packung anzubieten, beispielsweise solche zur Tages- und Nachtpflege, solche mit unterschiedlichen Farben, Düften, unterschiedlich starken Lichtschutzfaktoren oder unterschiedlichen Wirkstoffen. Es ist bei einer solchen Verwendung besonders vorteilhaft, die Kugeln unterschiedlicher Zusammensetzung durch unterschiedliche Form- und/oder Farbgebung für den Anwender unterscheidbar zu machen.

Nicht zuletzt ist die Verwendung der erfindungsgemäßen kosmetischen und/oder dermatologischen Zubereitungen zur Prophylaxe und Behandlung fettiger Haut sowie zur Prophylaxe und Behandlung von unreiner Haut sowie von Cellulite erfindungsgemäß.

Die Herstellung der erfindungsgemäßen kugelförmigen Zubereitungen wird nachfolgend beispielhaft beschrieben und besteht zunächst aus der Herstellung einer W/O-Emulsion aus den erfindungswesentlichen Bestandteilen mit nachfolgender Anwendung formgebender Verfahren.

Die Herstellung von Emulsionen aus nicht miteinander mischbaren Lipid- und Wasserphasen ist seit langem bekannt und erfolgt in der Regel durch Eintragen mechanischer Energie durch Verwendung geeigneter Rührwerke und Homogenisatoren. Da die erfindungsgemäßen Emulsionen bei Raumtemperatur fest sind, müssen alle Arbeitsschritte (Zugabe und Vermischen der Inhaltsstoffe, Homogenisieren, eventuelles Entschäumen durch Anlegen von Vakuum) bei Temperaturen oberhalb des Schmelzpunktes der Lipidphase durchgeführt werden müssen. In der Regel ist es nicht von Bedeutung, ob zuerst die Lipid- oder zuerst die Wasserphase zugegeben wird. Bei Verwendung nicht optimierter Anlagen und Formeln kann es sich aber für die Herstellung einer W/O-Emulsion als vorteilhaft herausstellen, die Lipidphase vorzulegen.

So kann die als Beispiel 1 beschriebene Rezeptur auf folgende Weise hergestellt werden: Ceresin, Methylpalmitat, Polyethylen, PEG-45/Dodecyl Glycol Copolymer, Polyglyceryl-3 Diisostearate, Dimethicon, Phenoxyethanol und Hexamidine Diisethionate werden unter Rühren aufgeschmolzen und auf 70°C erhitzt (Lipidphase).
Na-EDTA, alpha-Glycosylrutin, Glycerin, Ascorbylpalmitat, Farbstoff, und Zitronensäure werden in Wasser gelöst und die Lösung auf 70°C erhitzt (Wasserphase).
Die Wasserphase wird sodann unter Rühren in die Lipidphase gegeben, wodurch sich eine W/O-Emulsion bildet. Diese kühlt man unter Rühren weiter ab und gibt bei ca. 50°C das Parfum zu. Bei ca. 45°C wird das Rühren eingestellt und die Emulsion kühlt stehend weiter aus, bis sie schließlich fest wird. Dies ist unter Umständen erst nach längerem Stehen bei Raumtemperatur der Fall, da die erfindungsgemäßen Emulsionen mitunter Tendenzen zum Ausbilden einer unterkühlten Schmelze zeigen.

Die Herstellung der übrigen Beispiele erfolgt entsprechend.

Zur Lagerung und/oder dem Transport,kann die so hergestellte Emulsion beliebig oft erstarrt und wieder aufgeschmolzen werden. Zur Formung der erfindungsgemäßen Kugeln wird die Emulsion entweder oberhalb ihres Schmelzpunktes in entsprechende Formen gegossen und erstarrt dort, oder sie wird unterhalb ihres Schmelzpunktes unter Druck in Formen gepresst.

Die erfindungsgemäße Zubereitung weist verbesserte sensorische Eigenschaften auf, die mit wachshaltigen Zubereitungen aus dem Stand der Technik nicht zu erwarten sind. Es wurde eine verbesserte Verteilbarkeit, Einzugsvermögen, Konsistenz, Hautglättung und verminderte Klebrigkeit festgestellt. Bezüglich geeigneter Methoden zur Bestimmung dieser Parameter kann auf das Wissen des Fachmannes verwiesen werden.

Die Größenangaben wie beispielsweise der Durchmesser der Kugeln sind zu verstehen als Durchmesser in Richtung der Längsausdehnung der Kugeln.

Nachfolgende Beispiele erläutern die erfindungsgemäßen Zubereitungen. Die % Angaben beziehen sich soweit nichts anderes angegeben auf die Gesamtmasse der Zubereitungen.

### Beispiel 1

| | |
|---|---|
| Ceresin | 6% |
| Methylpalmitat | 21% |
| Polyethylen | 3% |
| PEG-45/Dodecyl Glycol Copolymer | 2,5% |
| Polyglyceryl-3 Diisostearate | 1,5% |
| Simethicon | 0,5 |
| Phenoxyethanol | 0,5% |
| Hexamidine Diisethionate | 0,08% |
| EDTA, Na-Salz | 0,2% |
| alpha-Glycosylrutin | 0,2% |
| Glycerin | 5% |
| Ascorbylpalmitat | 0,1 |
| Farbstoff | q.s. |
| Zitronensäure | q.s. |
| Parfum | q.s. |
| Wasser | ad 100 |

### Beispiel 2

| | |
|---|---|
| Ozokerit | 3% |
| Shea Butter | 21% |
| Polyethylen | 3% |
| PEG-45/Dodecyl Glycol Copolymer | 1% |
| Polyglyceryl-3 Diisostearate | 1% |
| Bis-Ethylhexyloxyphenol Methoxyphenyl Triazine | 2% |
| Octylmethoxycinnamat | 5% |
| Simethicon | 0,5 |
| Phenoxyethanol | 0,5% |
| Parabene | 0,08% |
| Harnstoff | 3% |
| Glycerin | 6% |
| Milchsäure | q.s. |
| Farbstoff | q.s. |
| Parfum | q.s. |
| Wasser | ad 100 |

### Beispiel 3

| | |
|---|---|
| hydrierte Kokosglyceride | 10% |
| Caprylyl/Caprinyltriglyceride | 25% |
| Polyethylen | 3% |
| Eucerit | 3,0% |
| TiO₂ | 2% |
| Aerosil R 812 | 1% |
| Dekaben LMB | 0,5% |
| Phenoxyethanol | 0,3% |
| Ubichinon | 0,1% |
| Butylenglycol | 3,0% |
| Panthenol | 3,0% |
| Glycerin | 4% |
| Zitronensäure | q.s. |
| NaOH | q.s. |
| Parfum | q.s. |
| Wasser | ad 100 |

### Beispiel 4

| | |
|---|---|
| Myristylmyristat | 15% |
| Cetylrhicinoleat | 20% |
| Ceresin | 5% |
| Polyglyceryl-3 Diisostearate | 1,5% |
| Polyglyceryl-2 Dipolyhydroxystearate | 1,5% |
| Mica | 2% |
| DMDM Hydantoin | 0,2% |
| Phenoxyethanol | 0,4% |
| Kreatin | 0,3% |
| Kreatinin | 0,1% |
| Niacinamid | 0,2% |
| Glycerin | 5% |
| Parfum | q.s. |
| Wasser | ad 100 |

### Beispiel 5

| | |
|---|---|
| Paraffinum liquidum | 15% |
| hydriertes Pflanzenfett | 15% |
| Cera Microcristallina | 10% |
| Eucerit | 1% |
| Polyglyceryl-2 Dipolyhydroxystearate | 1,5% |
| Nylon | 2% |
| Magnesiumstearat | 0,5% |
| Ethylhexyloxyglycerin | 0,5% |
| Parabene | 0,6% |
| Phenoxyethanol | 0,5% |
| Retinylpalmitat | 0,2% |
| Panthenol | 1% |
| Glycerin | 5% |
| Parfum | q.s. |
| Wasser | ad 100 |

### Beispiel 6

| | |
|---|---|
| Paraffinum liquidum | 15% |
| hydriertes Pflanzenfett | 15% |
| Cera Microcristallina | 10% |
| Polyglyceryl-3 Diisostearate | 1% |
| PEG-45/Dodecyl Glycol Copolymer | 2% |
| Distärkeoctenylsuccinat | 1% |
| Magnesiumstearat | 0,5% |
| Methylpropandiol | 0,5% |
| Parabene | 0,3% |
| Creatin | 0,2% |
| Phenoxyethanol | 0,3% |
| Tocopherylacetat | 0,5% |
| Glycerin | 5% |
| Eisenoxid-Pigmente | 1% |
| Parfum | q.s. |
| Wasser | ad 100 |

## Patentansprüche

1. Kosmetische und/oder dermatologische Zubereitung auf Basis einer W/O-Emulsion in Form von bis zu einer Temperatur von 35°C festen, halbfesten und/oder formstabilen Kugeln oder Sphäroiden mit einem Volumen von 0,1 bis 20 ml enthaltend im wesentlichen Wachse, Lipide, Emulgatoren, natürliche und/oder synthetische Polymere und/oder Mischungen daraus, **dadurch gekennzeichnet, dass** die Zubereitung aus einer formstabilen Lipid-/Emulgatormischung besteht, die dispergiertes Wasser mit einer Tröpfchengröße unter 50 Mikrometer enthält.

2. Zubereitung nach Anspruch 1, **dadurch gekennzeichnet, dass** die Wachse aus der Gruppe Cetylpalmitat, Cetylrhicinoleat, Bienenwachs, hydrierte Kokosglyceride, Methylpalmitat, Candelillawachs, Carnaubawachs, Paraffinwachs, Ceresin, Ozokerit, Myristylmyristat, Tripalmitin, Tribehenin, Glycerylpalmitostearat, hydriertes Rapsöl und/oder C15-C40 Alkylstearylstearat gewählt werden.

3. Zubereitung nach einem der Ansprüche 1 oder 2, **dadurch gekennzeichnet, dass** die Zubereitung Wachse, gewählt aus mikrokristallinen Wachsen, Paraffinwachsen, Esterwachsen, Glyceridwachsen, sowie Fettalkoholen und festen Emulgatoren und deren Mischungen enthält.

4. Zubereitung nach einem der vorstehenden Ansprüche, **dadurch gekennzeichnet, dass** sie einen durchschnittlichen Durchmesser von 3 bis 40 mm aufweist.

5. Zubereitung nach einem der vorstehenden Ansprüche, **dadurch gekennzeichnet, dass** zusätzlich Hilfsstoffe, UV-Filter, Pigmente. Wirkstoffe, Farbstoffe, Sensorikadditive, Verdicker, Gelbildner, Konservierungsmittel, Antioxidantien, Komplexbildner, Geschmacksstoffe, Vergällungsmittel, Wirkstoffe und/oder Parfum enthalten sind.

6. Zubereitung nach einem der vorstehenden Ansprüche, **dadurch gekennzeichnet, dass** die einzelnen kugelförmigen Zubereitungen von einer äußeren Verpackungshülle umgeben sind.

7. Zubereitung nach einem der vorstehenden Ansprüche, **dadurch gekennzeichnet, dass** die Kugelförmigen Zubereitungen einzeln oder zu mehreren in einer Packung aus Papier, Metall oder Kunststoff verpackt sind.

8. Zubereitung nach einem der vorstehenden Ansprüche, **dadurch gekennzeichnet, dass** die kugelförmigen Zubereitungen einzeln oder zu mehreren in einer Blisterverpackung aus Papier, Metall oder Plastik verpackt sind.

9. Zubereitung nach einem der vorstehenden Ansprüche in Kombination mit einer Blisterverpackung zur vorportionierten Einzelentnahme der kugelförmigen Zubereitung.

10. Verwendung der Zubereitung nach einem der vorstehenden Ansprüche zur topischen Anwendung auf Haut und/oder Haaren.

11. Verwendung der Zubereitung nach Anspruch 10 zur rückstandsfreien Aufbringung von Kosmetika.

12. Verwendung der Zubereitung nach Anspruch 10 oder 11 zur Einmalapplikation.

13. Verfahren zur Applikation kosmetischer und/oder dermatologischer Zubereitungen nach einem der Ansprüche 1 bis 9 zur topischen Anwendung, indem die Zubereitungen beim Verreiben und/oder Verteilen auf der Haut und/oder dem Haar
- schmelzen und/oder
- aufgrund von Scherkräften völlig oder teilweise flüssig werden und/oder
- sich in den Hautsebumlipiden ganz oder teilweise auflösen.

14. Verfahren zur Applikation und/oder dermatologischer Zubereitungen nach Anspruch 13 indem eine oder mehrere kugelförmige Zubereitung aus einer Verpackung entnommen und durch Reiben auf der Haut und/oder im Haar verteilt werden.

15. Kombination bestehend aus mehreren kugelförmigen Zubereitungen nach einem der Ansprüche 1 bis 9, **dadurch gekennzeichnet, dass** mindestens zwei der kugelförmigen Zubereitungen sich in ihrem Aussehen unterscheiden, unterschiedliche Inhaltsstoffe aufweisen und/oder verschiedenen Zwecken dienen.

## Claims

1. Cosmetic and/or dermatological preparation based on a W/O emulsion in the form of spheres or spheroids which are solid, semisolid and/or dimensionally stable up to a temperature of 35°C, have a volume of from 0.1 to 20 ml and comprise essentially waxes, lipids, emulsifiers, natural and/or synthetic polymers and/or mixtures thereof, **characterized in that** the preparation consists of a dimensionally stable lipid/emulsifier mixture which comprises dispersed water with a droplet size below 50 micrometers.

2. Preparation according to Claim 1, **characterized in that** the waxes are chosen from the group consisting of cetyl palmitate, cetyl ricinoleate, beeswax, hydrogenated cocoglycerides, methyl palmitate, candelilla wax, carnauba wax, paraffin wax, ceresine, ozokerite, myristyl myristate, tripalmitin, tribehenin, glyceryl palmitostearate, hydrogenated rapeseed oil and/or C15-C40 alkylstearyl stearate.

3. Preparation according to one of Claims 1 and 2, **characterized in that** the preparation comprises waxes chosen from microcrystalline waxes, paraffin waxes, ester waxes, glyceride waxes, and fatty alcohols and solid emulsifiers and mixtures thereof.

4. Preparation according to one of the preceding claims, **characterized in that** it has an average diameter of from 3 to 40 mm.

5. Preparation according to one of the preceding claims, **characterized in that** auxiliaries, UV filters, pigments, active ingredients, dyes, sensory additives, thickeners, gel formers, preservatives, antioxidants, complexing agents, flavorings, denaturants, active ingredients and/or perfume are additionally present.

6. Preparation according to one of the preceding claims, **characterized in that** the individual spherical preparations are surrounded by an outer packaging wrapper.

7. Preparation according to one of the preceding claims, **characterized in that** the spherical preparations are packaged individually or in multiples in a packaging made of paper, metal or plastic.

8. Preparation according to one of the preceding claims, **characterized in that** the spherical preparations are packaged individually or in multiples in a blister pack made of paper, metal or plastic.

9. Preparation according to one of the preceding claims in combination with a blister pack for the preportioned individual removal of the spherical preparation.

10. Use of the preparation according to one of the preceding claims for topical use on skin and/or hair.

11. Use of the preparation according to Claim 10 for the residue-free application of cosmetics.

12. Use of the preparation according to Claim 10 or 11 for single application.

13. Method of applying cosmetic and/or dermatological preparations according to one of Claims 1 to 9 for topical application in which the preparations, upon rubbing and/or distributing on the skin and/or the hair,
- melt and/or
- become completely or partially liquid due to shear forces and/or
- completely or partially dissolve in the skin sebum lipids.

14. Method of applying cosmetic and/or dermatological preparations according Lo Claim 16 by removing one or more spherical preparation from a pack and distributing it on the skin and/or in the hair by rubbing.

15. Combination consisting of a plurality of spherical preparations according to one of Claims 1 to 9, **characterized in that** at least two of the spherical preparations differ in their appearance, have different ingredients and/or serve different purposes.

## Revendications

1. Composition cosmétique et/ou dermatologique à base d'une émulsion E/H sous forme de billes ou de sphéroïdes solides, semi-solides et/ou de forme stable jusqu'à une température de 35°C, présentant un volume de 0,1 à 20 ml, contenant essentiellement des cires, des lipides, des émulsifiants, des polymères naturels et/ou synthétiques et/ou leurs mélanges, **caractérisée en ce que** la composition est constituée par un mélange lipides/émulsifiant de forme stable qui contient de l'eau dispersée d'une grosseur de particules inférieure à 50 micromètres.

2. Composition selon la revendication 1, **caractérisée en ce que** les cires sont choisies dans le groupe formé par le palmitate de cétyle, le ricinoléate de cétyle, la cire d'abeille, les glycérides de coco hydrogénés, le palmitate de méthyle, la cire de candelilla, la cire de caroube, la cire de paraffine, la cérésine, l'ozokérite, le myristate de myristyle, la tripalmitine, la tribéhénine, le palmitostéarate de glycéryle, l'huile de colza hydrogénée et/ou le stéarate de C₁₅-C₄₀-alkylstéaryle.

3. Composition selon l'une quelconque des revendications 1 ou 2, **caractérisée en ce que** la composition contient des cires, choisies parmi les cires microcristallines, les cires de paraffine, les cires d'ester, les cires de glycéride, ainsi que des alcools gras et des émulsifiants solides et leurs mélanges.

4. Composition selon l'une quelconque des revendications précédentes, **caractérisée en ce qu'**elle présente un diamètre moyen de 3 à 40 mm.

5. Composition selon l'une quelconque des revendications précédentes, **caractérisée en ce qu'**elle contient en outre des adjuvants, des filtres UV, des pigments, des substances actives, des colorants, des additifs améliorant le toucher, des épaississants, des gélifiants, des conservateurs, des antioxydants, des complexants, des substances gustatives, des dénaturants, des substances actives et/ou des parfums.

6. Composition selon l'une quelconque des revendications précédentes, **caractérisée en ce que** les différentes compositions sphériques individuelles sont entourées par une enveloppe d'emballage extérieure.

7. Composition selon l'une quelconque des revendications précédentes, **caractérisée en ce que** les compositions sphériques sont emballées seules ou à plusieurs dans un paquet en papier, métal ou matériau synthétique.

8. Composition selon l'une quelconque des revendications précédentes, **caractérisée en ce que** les compositions sphériques sont emballées seules ou à plusieurs dans un emballage à blister en papier, métal ou plastique.

9. Composition selon l'une quelconque des revendications précédentes, en combinaison avec un emballage à blister pour le prélèvement individuel portionné au préalable de la composition sphérique.

10. Utilisation de la composition selon l'une quelconque des revendications précédentes pour l'utilisation topique sur la peau et/ou les cheveux.

11. Utilisation de la composition selon la revendication 10 pour l'application sans résidus de produits cosmétiques.

12. Utilisation de la composition selon la revendication 10 ou 11 pour une application unique.

13. Utilisation pour l'application de compositions cosmétiques et/ou dermatologiques selon l'une quelconque des revendications 1 à 9 pour l'application topique, en ce que les compositions, lorsqu'on les frotte et/ou répartit sur la peau et/ou les cheveux
- fondent et/ou
- deviennent totalement ou partiellement liquides en raison de forces de cisaillement et/ou
- se dissolvent totalement ou partiellement dans les lipides du sébum de la peau.

14. Procédé pour l'application des compositions cosmétiques et/ou dermatologiques selon la revendication 13 en ce qu'une ou plusieurs compositions sphériques sont prélevées d'un emballage et réparties par frottement sur la peau et/ou dans les cheveux.

15. Combinaison constituée par plusieurs compositions sphériques selon l'une quelconque des revendications 1 à 9, **caractérisée en ce qu'**au moins deux des compositions sphériques se distinguent par leur aspect, présentent des constituants différents et/ou servent à des fins différentes.
